(19) Europäisches Patentamt
European Patent Office
Office européen des brevets

(11) Publication number: **0 026 034**
**B1**

(12) **EUROPEAN PATENT SPECIFICATION**

(45) Date of publication of patent specification: **23.03.83**

(51) Int. Cl.³: **C 07 D 231/38, A 01 N 43/56**

(21) Application number: **80302652.5**

(22) Date of filing: **04.08.80**

(54) **5-Amino-4-cyano-1-(2,4,6-trichlorophenyl)pyrazole, process for its preparation, herbicidal compositions containing it and their use.**

(30) Priority: **23.08.79 GB 7929318**
**15.12.79 GB 7943285**

(43) Date of publication of application:
**01.04.81 Bulletin 81/13**

(45) Publication of the grant of the patent:
**23.03.83 Bulletin 83/12**

(84) Designated Contracting States:
**AT BE CH DE FR GB IT LI LU NL SE**

(56) References cited:
**CH - A - 365 730**
**US - A - 3 760 083**

(73) Proprietor: **FBC LIMITED**
**Hauxton**
**Cambridge CB2 5HU (GB)**

(72) Inventor: **Ralph, Philip David**
**28 Bina Gardens**
**London, S.W.5 (GB)**
Inventor: **Ayres, Robert John**
**9 Greenfield Close**
**Stapleford Cambridge (GB)**

(74) Representative: **Richer, David Leonard Industrial Property Department FBC Limited et al,**
**Chesterford Park Research Station**
**Saffron Walden, Essex CB10 1XL (GB)**

**EP 0 026 034 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# 5-Amino-4-cyano-1-(2,4,6-trichlorophenyl)pyrazole, process for its preparation, herbicidal compositions containing it and their use

The present invention relates to a new compound, its preparation, plant physiologically active compositions containing it and its use as a herbicide and plant growth regulant.

Accordingly the invention provides a compound which is 5-amino-4-cyano-1-(2,4,6-trichlorophenyl)pyrazole or a salt thereof.

The invention provides also a process for preparing the compound, which process comprises reacting 2,4,6-trichlorophenylhydrazine with a lower alkoxymethylenemalononitrile whose lower alkoxy group contains 1—4 carbon atoms.

In addition, the invention provides a plant physiologically active composition containing the compound, in particular together with at least one material selected from surface active agents, carriers, antidotes, fertilizers and other plant growth regulants and pesticides.

The invention also provides a method of combating weeds at a locus infested or liable to be infested with them, or of regulating the growth of a plant at a locus at which the plant is growing or is to grow, which method comprises applying to the locus a weed-combating or plant growth regulant amount of the compound.

The compound has a great effect on plant physiology, and is useful as a herbicide and plant growth regulant. Moreover this activity could not be derived from the prior art, see for example Swiss Patent No. 365730 which discloses certain pyrazole triazines having pharmacological activity. As intermediates in their preparation, there are mentioned certain 2-phenyl-3-amino-4-cyanopyrazoles, but there is no indication that they possess any herbicidal activity. United States Patent No. 3760083 describes the compound 5-amino-3-ethyl-1-(3-trifluorophenyl)-4-pyrazolecarboxamide which is stated to be pharmacologically active.

The activity of the claimed compound is outstandingly high compared to that of compounds closely related in chemical structure. It is outstandingly useful as a herbicide, especially as a selective herbicide.

The compound can be prepared by reacting 2,4,6-trichlorophenylhydrazine with the lower alkoxymethylenemalononitrile. The lower alkoxymethylenemalononitrile is suitably ethoxymethylenemalononitrile, of formula $C_2H_5OCH{=}C(CN)_2$. The reaction is usually conducted in an inert solvent, e.g. an alcohol. The reaction may be conducted at a temperature for example of 0—150°C.

The compound is preferably employed as the pyrazole itself, though salts can be employed, e.g. salts with strong acids such as hydrochloric, sulphuric or nitric acid. Salts may be formed from the non-salt form in ways conventional for other salts, and may be converted to the non-salt form in ways conventional for other salts. Thus, a salt may be formed by reaction of the pyrazole with an acid, and the salt may be converted to the pyrazole by reaction with a base.

The compound can be formulated into plant physiologically active compositions, e.g. herbicidal compositions, in the usual way. They can be prepared by admixing the ingredients. Usually the compositions are initially produced in the form of concentrates, e.g. containing 0.5—80%, for example 10—50%, of the present compound, and these are diluted with water or hydrocarbon, usually water, for application by spraying, generally such that the concentration of the compound is 0.05—5%. Percentages, ratios and parts in this specification are by weight unless otherwise indicated.

The compositions normally contain a surface active agent and/or a carrier.

The carrier may be a liquid, e.g. water (e.g. water used to dilute a concentrate for application). If water is employed as carrier in a concentrate, an organic solvent may also be present as carrier, though this is not usually employed. A surface active agent may advantageously be present.

The carrier may be a liquid other than water, for example an organic solvent, usually a water-immiscible solvent, e.g. a hydrocarbon which boils within the range 130—270°C, in which the compound is dissolved or suspended. A concentrate containing an organic solvent suitably also contains a surface active agent so that the concentrate acts as a self-emulsifiable oil on admixture with water. The liquid may be a water-miscible solvent, e.g. 2-methoxyethanol, methanol, propylene glycol, diethylene glycol, diethylene glycol monoethyl ether, formamide or methylformamide.

The carrier is preferably, however, a solid, which may be finely divided. Examples of suitable solids are limestone, clays, sand, mica, chalk, attapulgite, diatomite, perlite, sepiolite, silicas, silicates, lignosulphonates and solid fertilizers. The carrier can be of natural or synthetic origin or can be a modified natural material.

Wettable powders soluble or dispersible in water may be formed by admixing the compound in particulate form with a particulate carrier or spraying molten compound on to the particulate carrier, admixing a wetting agent and a dispersing agent, and finely grinding the whole powder mixture.

An aerosol composition may be formed by admixing the compound with a propellant, e.g. a polyhalogenated alkane such as dichlorodifluoromethane, and suitably also with a solvent.

A flowable suspension concentrate may be formed by grinding the compound with water, a wetting agent and a suspending agent.

A flowable suspension concentrate wherein the carrier is a hydrocarbon which boils within the range 130—270°C rather than water may be formed.

Thus the present composition can for example be solid (e.g. dust or granules) and contain a solid carrier, or liquid (e.g. an emulsifiable concentrate) and contain a liquid carrier which is a hydrocarbon which boils within the range 130—270°C.

The term 'surface-active agent' is used in the broad sense to include materials variously called emulsifying agents, dispersing agents and wetting agents. Such agents are well known in the art.

The surface active agents used may comprise anionic surface active agents, for example mono- or di-esters of phosphoric acid with fatty alcohol ethoxylates or with alkylphenol ethoxylates, salts of such esters, fatty alcohol sulphates such as sodium dodecyl sulphate, sodium octadecyl sulphate or sodium cetyl sulphate, ethoxylated fatty alcohol sulphates, ethoxylated alkylphenol sulphates, lignin sulphonates, petroleum sulphonates, alkylaryl sulphonates such as alkyl-benzene sulphonates or lower alkylnaphthalene sulphonates, e.g. butyl-naphthalene sulphonate, salts of sulphonated naphthalene-formaldehyde condensates, salts of sulphonated phenol-formaldehyde condensates, or more complex sulphonates such as the amide sulphonates, e.g. the sulphonated condensation product of oleic acid and N-methyl taurine or the dialkyl sulphosuccinates, e.g. the sodium sulphonate of dioctyl succinate.

The surface active agents may also comprise non-ionic agents, for example condensation products of fatty acid esters, fatty alcohols, fatty acid amides or fatty-, alkyl- or alkenyl-substituted phenols with ethylene oxide, fatty esters of polyhydric alcohol ethers, e.g. sorbitan fatty acid esters, condensation products of such esters with ethylene oxide, e.g. polyoxyethylene sorbitan fatty acid esters, block copolymers of ethylene oxide and propylene oxide, acetylenic glycols such as 2,4,7,9-tetramethyl-5-decyn-4,7-diol, or ethoxylated acetylenic glycols.

The surface active agents may also comprise cationic agents, for example alkyl- and/or aryl-substituted quaternary ammonium compounds such as cetyl trimethylammonium bromide or ethoxylated tertiary fatty amines.

Preferred surface active agents include ethoxylated fatty alcohol sulphates, lignin sulphonates, alkyl-aryl sulphonates, salts of sulphonated naphthalene-formaldehyde condensates, salts of sulphonated phenol-formaldehyde condensates, dialkyl sulphosuccinates, alkyl phenol ethoxylates, and fatty alkyl ethoxylates.

Non-ionic surface active agents are preferred.

The compound may be in admixture with non-phytotoxic oils, e.g. Agri-Oil Plus, Sun Oil 11E or Fyzol 11E.

The compound may be employed in association with a herbicidal antidote (a substance having the property of improving the safety of a herbicide to a crop), e.g. N,N-diallyl-2,2-dichloroacetamide, 4'-chloro-2-(hydroxyimino)-acetanilide, 1,8-naphthalic anhydride, $\alpha$-(cyanomethoximino)-benzeneaceto-nitrile or 2,2-dimethyl-3-dichloroacetyloxazolidine. Although the antidote may be applied in admixture with active compound, it is preferably applied separately, and especially as a treatment for crop seeds. The ratio by weight of herbicide to antidote is preferably from 1:4 to 4:1.

The compound may be used in sequence or admixture with a fertilizer.

The compound may be used in sequence or admixture with another plant growth regulant or pesticide, e.g. herbicide, fungicide or insecticide. The compound is preferably employed in admixture or sequence, especially admixture, with another herbicide. The invention provides a one pack presentation, in which the compound is already mixed with other pesticide or plant growth regulant, and also a single package designed to hold the compound and other pesticide or plant growth regulant in separate containers, for mixing, e.g. in a spray tank, for application.

The other herbicide may be for example one or more of a phenoxyaliphatic acid, substituted urea, triazine, phenol, nitrile, bipyridylium compound, substituted benzoic acid, halogenated aliphatic acid, carbamate, thiocarbamate, chloroacetamide, diazine or arsenic herbicide.

The phenoxyaliphatic acid generally comprises alkyl and/or halogen substituted phenoxyaliphatic acids, and their salts, for example alkali metal, amine and alkanolamine salts, and functional derivatives, for example esters and amides. These compounds may be of activity such that they are recognised as commercial herbicides, or may be of only slight herbicidal activity. Examples of the substituted phenoxyaliphatic acids which may be mentioned include 2,4-dichlorophenoxyacetic acid, 2-(2,4-dichlorophenoxy)propionic acid, 2-methyl-4-chlorophenoxyacetic acid, 2,4,5-trichlorophenoxyacetic acid, gamma-2,4-dichlorophenoxybutyric acid, gamma-2-methyl-4-chlorophenoxybutyric acid, alpha-2-methyl-4-chlorophenoxypropionic acid, 2-(4-[2,4-dichlorophenoxy]phenoxy)propionic acid and 2-(4-[4-chlorophenoxy]phenoxy)propionic acid.

The substituted urea generally comprises a tri- or tetra-substituted urea such as N'-(3-chloro-4-methoxyphenyl)-N,N-dimethylurea, N'-(3-chloro-4-methylphenyl)-N,N-dimethylurea, N'-parachloro-phenyl-N,N-dimethylurea, N-butyl-N'-(3,4-dichlorophenyl)-N-methylurea, N'-parachlorophenyl-O,N,N-trimethylisourea, N'-*p*-chlorophenyl-N-methoxy-N-methylurea, N,N-dimethyl-N'-phenylurea, N-(4-bromophenyl)-N'-methoxy-N'-methylurea, N-(2-benzothiazolyl)-N'-methylurea, N-benzothiazol-2-yl-N,N'-dimethylurea, N,N-dimethyl-N'-(4-[1-methylethyl]phenyl)urea, N'-(3,4-dichlorophenyl)-N-methoxy-N-methylurea or N,N-dimethyl-N'-[3-(trifluoromethyl)phenyl]urea.

The triazine herbicide generally comprises 2-chloro-4-(1-cyano-1-methylamino)-6-ethylamino-1,3,5-triazine or 2-isopropylamino-4-(3-methoxypropylamino)-6-methylthio-1,3,5-triazine or a compound of the formula:—

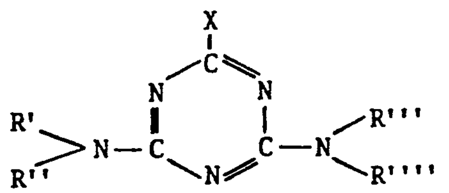

where X is a halogen atom, alkoxy or alkylthio, R′ and R‴ are the same or different and are hydrogen or alkyl and R″ and R⁗ are the same or different alkyl groups, such as 2-chloro-4,6-bisethylamino-1,3,5-triazine, 2-chloro-4-ethylamino-6-diethylamino-1,3,5-triazine, 2-chloro-4-ethylamino-6-isopropylamino-1,3,5-triazine or 2,4-bis(isopropylamino)-6-methylthio-1,3,5-triazine.

The phenol herbicide generally comprises 4,6-dinitro-*o*-cresol, 4,6-dinitro-2-sec-butylphenol or pentachlorophenol. The nitrile herbicide generally comprises 3,5-diiodo-4-hydroxybenzonitrile, 3,5-dibromo-4-hydroxybenzonitrile or 2,6-dichlorobenzonitrile. The bipyridylium herbicide generally comprises 1,1′-dimethyl-4,4′-bipyridylium dichloride or 1,1′-ethylene-2,2′-bipyridylium dibromide. The substituted benzoic acid herbicide generally comprises 2,3,6-trichlorobenzoic acid, 2-methoxy-3,6-dichlorobenzoic acid, N-(1,1-dimethylpropynyl)-3,5-dichlorobenzamide or methyl 5-(2,4-dichlorophenoxy)-2-nitrobenzoate. The halogenated aliphatic acid herbicide generally comprises trichloroacetic acid or 2,2-dichloropropionic acid. The carbamate herbicide generally comprises isopropyl N-(3-chlorophenyl)carbamate, 4-chloro-2-butynyl N-(3-chlorophenyl)carbamate, methyl 3-(*m*-tolylcarbamoyloxy)phenylcarbamate or D-N-ethyl-2-(phenylcarbamoyloxy)propionamide. The thiocarbamate herbicide generally comprises S-ethyl N,N-dipropylthiocarbamate, S-ethyl N,N-diisobutylthiocarbamate, S-(2,3-dichloroallyl) N,N-diisopropylthiocarbamate, S-ethyl N-ethyl-N-cyclohexylthiocarbamate, S-propyl butylethylthiocarbamate, S-(2,3,3-trichloroallyl) N,N-diisopropylthiocarbamate or S-(4-chlorobenzyl) N,N-diethylthiocarbamate. The chloroacetamide herbicide generally comprises N,N-diallyl-2-chloroacetamide or N-isopropyl-2-chloroacetanilide. The diazine herbicide generally comprises 5-bromo-6-methyl-3-*sec*-butyluracil, 3-cyclohexyl-5,6-trimethyleneuracil, 5-amino-4-chloro-2-phenyl-3-pyridazinone or 1,2-dihydropyridazine-3,6-dione. The arsenic herbicide generally comprises a salt of methane arsenic acid or cacodylic acid. Other herbicides which may be used as the second herbicide include 1,2-dimethyl-3,4-diphenylpyrazolium ion, ethyl N-benzoyl-N-(3,4-dichlorophenyl)alanine, N-iso-butyl-2-oxo-1-imidazilidinecarboxamide, aminotriazole, 2,3-dichloro-1,4-naphthoquinone, 4-amino-3,5,6-trichloropicolinic acid, N,N-dimethyl-2,2-diphenylacetamide, 2,6-dinitro-N,N-dipropyl-4-trifluoromethylaniline, N-butyl-N-ethyl-2,6-dinitro-4-trifluoromethylaniline, S,S,S-tributyl phosphorotrithioate, 2-ethoxy-2,3-dihydro-3,3-dimethyl-5-benzofuranyl methylsulphonate, 4-chloro-2-oxobenzothiazolin-3-yl acetic acid, 3-isopropyl-(1H)-benzo-2,1,3-thiadiazin-4-one 2,2-dioxide, 3,5-dibromo-4-hydroxybenzaldehyde 2,4-dinitrophenyloxime, methyl 2-chloro-3-(4-chlorophenyl)propionate, 2-chloroethyltrimethylammonium chloride, isopropyl 2-(N-benzoyl-3-chloro-4-fluoroanilino)propionate, methyl(2-(N-benzoyl-3-chloro-4-fluoroanilino)propionate, 2-chloro-N-(1,3-dioxolan-2-ylmethyl)-2′,6′-dimethylacetanilide, 2-chloro-1-(3-ethoxy-4-nitrophenoxy)-4-trifluoromethylbenzene, methyl 2-(4-[2′,4′-dichlorophenoxy]phenoxy)propionate, isobutyl 2-(4-[4′-chlorophenoxy]phenoxy)propionate or N-[(1-ethylpropyl)-3,4-dimethyl-2,6-dinitrobenzenamine.

The other herbicide may be particularly a herbicide which has high activity against mayweed or chickweed. The other herbicide may be especially one or more of metoxuron (3-(3-chloro-4-methoxyphenyl)-1,1-dimethylurea), simazine (2-chloro-4,6-di(ethylamino)-1,3,5-triazine), trietazine (2-chloro-4-diethylamino-6-ethylamino-1,3,5-triazine), methoprotryne (2-isopropylamino-4-(3-methoxypropylamino)-6-methylthio-1,3,5-triazine), terbutryne (2-tertbutylamino-4-ethylamino-6-methylthio-1,3,5-triazine), bentazone (3-isopropyl-(1H)-benzo-2,1,3-thiadiazin-4-one 2,2-dioxide), ioxynil (4-hydroxy-3,5-diiodobenzonitrile), bromoxynil (3,5-dibromo-4-hydroxybenzonitrile), 3,6-dichloropicolinic acid, CMPP (2-(4-chloro-2-methylphenoxy)propanoic acid), linuron (3-(3,4-dichlorophenyl)-1-methoxy-1-methylurea), chlortoluron (3-(3-chloro-4-methylphenyl)-1,1-dimethylurea), isoproturon (3-(4-isopropylphenyl)-1,1-dimethylurea), thiobencarb (S-(4-chlorobenzyl) N,N-diethylthiocarbamate), bifenox (methyl 5-(2,4-dichlorophenoxy)-2-nitrobenzoate), pendimethalin (N-[(1-ethylpropyl)-3,4-dimethyl-2,6-dinitrobenzenamine]) and methabenzthiazuron (1-benzothiazol-2-yl-1,3-dimethylurea), for example a mixture of metoxuron and simazine, of CMPP and bentazone, of CMPP and ioxynil and/or bromoxynil, or of CMPP and 3,6-dichloropicolinic acid. For instance, for pre-emergence use, one may employ mixtures of the compound with trietazine, with chlortoluron, with isoproturon, with bifenox, with pendimethalin, with methabenzthiazuron, or with metoxuron and simazine. For post-emergence use, one may employ for instance mixtures of the compound with metoxuron and simazine, with methoprotryne, with terbutryne, with bentazone (e.g. mixed with CMPP), with CMPP (e.g. mixed with 3,6-dichloropicolinic acid, or with ioxynil and/or bromoxynil), or with linuron.

In a particularly preferred embodiment, the present compound is employed in combination with chlortoluron.

In another particularly preferred embodiment, the present compound is employed in combination with isoproturon.

In another particularly preferred embodiment, the present compound is employed in combination with terbutryne.

In another particularly preferred embodiment, the present compound is employed in combination with thiobencarb.

The ratio of present compound to other pesticide or plant growth regulant may vary over a wide range, depending for instance on the particular other material. In general, however, the ratio of present compound to other pesticide or plant growth regulant lies in the range 1:11 to 10:1, e.g. 1:10 to 10:1. In a particular embodiment, the ratio of present compound to other pesticide or plant growth regulant lies in the range 1:11 to 2:1.

The present compound is active against a wide range of weeds. It is highly active against

mayweed — *Matricaria inodora*
field pansy — *Viola arvensis*
black bindweed — *Polygonum convolvulus*
fathen — *Chenopodium album*
shepherds purse — *Capsella bursa pastoris*
annual nettle — *Urtica dioica*
field speedwell — *Veronica persica*
ivyleaved speedwell — *Veronica hederafolia,* and
cleavers — *Galium aparine.*

In the use of the present compound as a total herbicide, high rates of application, for example at least 10 kg per hectare, such as 10—25 kg per hectare, of the compound are usually required, unless it is mixed with other herbicides, in which case the rate can be reduced.

In the use of the present compound as a selective herbicide, the rate of application is usually much lower and may be for example $\frac{1}{4}$—10, e.g. $\frac{1}{4}$—2, kg per hectare.

In the use of the compound as a plant growth regulant, low rates of application are usually required, such as 0.1—4, e.g. 0.1—1, kg per hectare.

Usually the compound is applied to plants, the land or soil. The compound can be employed for pre-emergent or post-emergent use. The compound is preferably employed for selectively combating weeds at a locus at which a crop is growing, or less preferably, is to grow. The compound may be applied pre- or post-planting of the crop. The crop is preferably a food crop, e.g. a vegetable crop. The crop may be for instance a plantation crop or an ornamental crop. Preferably, the crop is maize, millet, legumes (e.g. field beans), sorghum, ryegrass, onions, oil seed rape, carrots, peanuts, swedes, cotton or a cereal crop. It is particularly preferred that the crop be a cereal crop, e.g. rice or oats and especially wheat or barley. In a preferred embodiment, the compound is employed post-emergence for the control of broad leaved weeds in a cereal crop.

The invention is illustrated by the following Examples. 'Compound' in the Tables in Examples 11—16 denotes 5-amino-4-cyano-1-(2,4,6-trichlorophenyl)pyrazole.

## Example 1

2,4,6-Trichlorophenylhydrazine (106 parts) and ethoxymethylenemalononitrile (92 parts) were dissolved in ethanol (2000 parts) and heated under reflux for one hour. After removing some red impurities by filtration, the solvent was evaporated and the residue recrystallised from a small amount of ethanol to give 5-amino-4-cyano-1-(2,4,6-trichlorophenyl)pyrazole (63 parts, 48% yield) as white crystals, melting point 195—197°C.

*Analysis:*

| | |
|---|---|
| Found: | C 41.5; H 1.70; Cl 36.9; N 19.2 |
| $C_{10}H_5Cl_3N_4$ requires: | C 41.8; H 1.75; Cl 37.0; N 19.5% |

## Example 2

The compound 5-amino-4-cyano-1-(2,4,6-trichlorophenyl)pyrazole was formulated as an attaclay/sand dust and incorporated into John Innes No I potting compost at a rate of 130 parts per million weight/volume of compound to soil and placed in anodised aluminium pans, 20 cm long × 10 cm wide × 5 cm deep. This rate is approximately equivalent to a soil surface application of 56 kg of compound per hectare cultivated to a depth of 5 cm. Seeds of peas (*Pisum sativum*), mustard (*Sinapis alba*), linseed (*Linum usitatissimum*), maize (*Zea mays*), oats (*Avena sativa*) and ryegrass (*Lolium* sp.) were then sown in the treated soil, one species to each pan, watered and kept in a controlled environment room (temperature 22°C, relative humidity 65—85%, 14 hours per day artificial illumination of 13,000 lux) for 21 days.

The plants were then assessed visually for any growth regulatory or herbicidal effect, differences from untreated controls being assessed on a scale from 0 to 100, in which 0 signifies no effect and 100 signifies complete suppression. The results are listed below:

| | Effect |
|---|---|
| Peas | 98 |
| Mustard | 100 |
| Linseed | 100 |
| Maize | 99 |
| Oats | 100 |
| Ryegrass | 100 |

Example 3

Seeds of the plant species listed below were sown in anodised aluminium pans, 19 cm long x 9.5 cm wide x 5 cm deep, containing John Innes No I potting compost. They were then watered and placed in a controlled environment room (22°C; 65—85% relative humidity; 14 hours per day artificial illumination, at 13,000 lux). Fourteen days after sowing, the seedlings received a foliar spray of the compound 5-amino-4-cyano-1-(2,4,6-trichlorophenyl)pyrazole formulated as a solution/suspension in 1:1 by volume aqueous acetone.

The concentration of the test compound and volume of application were adjusted so as to be equivalent to a rate of 2.8 kg of the compound in 450 litres per hectare. After 7 days growth in the controlled environment room, the plants were visually assessed for any herbicidal or growth regulant response. All differences from an untreated control were scored according to an index were 0 = no effect and 9 = complete kill. The results are shown in the following table:—

| Species | Effect |
|---|---|
| Peas (*Pisum sativum*) | 4 |
| Mustard (*Sinapis alba*) | 4 |
| Linseed (*Linum usitatissimum*) | 4 |
| Ryegrass (*Lolium perenne*) | 2 |
| Oats (*Avena sativa*) | 2 |
| Sugarbeet (*Beta vulgaris*) | 8 |

Example 4

The compound 5-amino-4-cyano-1-(2,4,6-trichlorophenyl)pyrazole was formulated as an attaclay/sand dust and incorporated into John Innes No I potting compost at a rate equivalent to 6.5 parts per million weight/volume of compound to soil and placed in anodised aluminium pans 20 cm long x 10 cm wide x 5 cm deep. This rate is approximately equivalent to a soil surface application of 2.8 kilograms of compound per hectare cultivated to a depth of 5 cm. Seeds of the species listed below were then sown in the treated soil, one species per pan, watered, and placed in a controlled environment room (temperature 22°C, relative humidity 65—86%, artificial illumination 14 hours per day of 17,000 lux).

After 21 days the plants were assessed visually for any growth regulatory or herbicidal effects. Differences from untreated controls were assessed on a scale from 0 to 100 in which 0 signifies no effect and 100 signifies complete suppression. The results are listed below:

| | | Effect |
|---|---|---|
| Chickweed | (*Stellaria media*) | 45 |
| Mustard | (*Sinapis alba*) | 98 |
| Cotton | (*Gossypium* spp.) | 65 |
| Tomato | (*Lycopersicon esculentum*) | 100 |
| Fathen | (*Chenopodium album*) | 100 |
| Carrot | (*Daucus carota*) | 90 |
| Wheat | (*Tritium aestivum*) | 93 |
| Barley | (*Hordeum vulgare*) | 70 |
| Wild Oats | (*Avena* spp.) | 85 |
| Blackgrass | (*Alopecurus myosuroides*) | 75 |
| Barnyardgrass | (*Echinochloa crus galli*) | 95 |
| Crabgrass | (*Digitaria sanguinalis*) | 95 |

Example 5

A 50% wettable powder was prepared by micronising the following:

| | |
|---|---|
| 5-amino-4-cyano-1-(2,4,6-trichlorophenyl)pyrazole | 50% |
| Arkopon T highly conc (sodium N-oleyl-N-methyltauride *ex* Hoechst) | 3% |
| Sopropon T 36 (sodium polycarboxylate *ex* Rhone-Poulenc) | 2% |
| China Clay | 45% |

Example 6

The composition of Example 5 was sprayed in an aqueous suspension at 200 litres of liquid per hectare, post-emergence in the greenhouse at the rate of 0.5 kg of the pyrazole compound per hectare onto the plant species listed in the Table below, which also records the herbicidal and plant growth regulant effect achieved, on a scale from 0 to 10 on which 0 represents no effect and 10 complete kill.

| Species | | | Effect |
|---|---|---|---|
| Winter Barley | — | *Hordeum vulgare* | 0.5 |
| Barnyardgrass | — | *Echinochloa crus-galli* | 6 |
| Crabgrass | — | *Digitaria sanguinalis* | 8 |
| Sprangletop | — | *Leptochloa dubium* | 10 |
| Pigweed | — | *Amaranthus retroflexus* | 10 |
| Ribwort | — | *Plantago lanceolata* | 10 |
| Speedwell | — | *Veronica persica* | 10 |
| Velvetleaf | — | *Abutilon threophrastii* | 10 |
| Mayweed | — | *Matricaria inodora* | 10 |
| Cleavers | — | *Galium aparine* | 9.5 |
| Pale Persicaria | — | *Polygonum lapathifolium* | 10 |

Example 7

The composition of Example 5 was sprayed in an aqueous suspension onto various field sites at the rate of 1 kg of the pyrazole compound per hectare pre-emergence where the crop species listed in the Table below had been sown. The mean herbicidal and plant growth regulant effect on the crop and weeds is recorded on a scale from 0 to 10 on which 0 represents no effect and 10 complete kill.

| Species | | | Effect |
|---|---|---|---|
| *Crops* | | | |
| Wheat | — | *Tritium aestivum* | 0.5 |
| Barley | — | *Hordeum vulgare* | 0.5 |
| Maize | — | *Zea mais* | 0.5 |
| Rice | — | *Oryza sativa* | 1 |
| Pearl Millet | — | *Pennisetum typhoideum* | 1 |
| Foxtail Millet | — | *Setaria italica* | 0.5 |
| Perennial Ryegrass | — | *Lolium perenne* | 1.5 |
| Navy Beans | — | *Phaseolus vulgaris* | 1 |
| Field Beans | — | *Vicia faba* | 0 |
| Cotton | — | *Gossypium spp* | 0 |
| Lentils | — | *Lens esculenta* | 1.5 |
| Peanuts | — | *Arachis hypogea* | 1 |
| *Weeds* | | | |
| Pigweed | — | *Amaranthus retroflexus* | 9.5 |
| Fat Hen | — | *Chenopodium album* | 7 |
| Chickweed | — | *Stellaria media* | 9.5 |
| Speedwell | — | *Veronica persica* | 9.5 |
| Mayweed | — | *Matricaria inodora* | 10 |
| Black Bindweed | — | *Polygonum convolvulus* | 6 |
| Field Pansy | — | *Viola arvensis* | 9 |

Example 8

The composition of Example 5 was sprayed in an aqueous suspension onto a field site in the United Kingdom at the rate of 0.5 kg of the pyrazole compound per hectare post-emergence of the crop species listed in the Table below, which also records the herbicidal and plant growth regulant effect on the crop and weeds assessed $3\frac{1}{2}$ weeks after the chemical treatment on a scale from 0 to 10 on which 0 represents no effect and 10 complete kill.

| Species | | | Effect |
|---|---|---|---|
| *Crops* | | | |
| Maize | — | *Zea mais* | 0.5 |
| Pearl Millet | — | *Pennisetum typhoideum* | 0 |
| Common Millet | — | *Panicum miliaceum* | 1 |
| Foxtail Millet | — | *Setaria italica* | 1.5 |
| Sorghum | — | *Sorghum vulgare* | 1 |
| Wheat — Sappo | — | *Triticum vulgare* | 1.5 |
| Barley — Proctor | — | *Hordeum vulgare* | 0.5 |
| Barley — Julia | — | *Hordeum vulgare* | 0.5 |
| Oats | — | *Avena sativa* | 0.5 |
| Rice — Bahilla | — | *Oryza sativa* | 1.5 |
| Rice — Ringo | — | *Oryza sativa* | 1.5 |
| Italian Ryegrass | — | *Lolium multiflorum* | 0.5 |
| Perennial Ryegrass — S23 | — | *Lolium perenne* | 1 |
| Perennial Ryegrass — Manhatten | — | *Lolium perenne* | 1 |
| Onion | — | *Allium cepa* | 0.5 |
| Oil Seed Rape | — | *Brassica napus* | 0 |
| Linseed | — | *Linum usitatissimum* | 0.5 |
| Swede | — | *Brassica campestris* | 1 |
| Lupins — Bitter | — | *Lupinus spp* | 1.5 |
| Lupins — Sweet | — | *Lupinus spp* | 1 |
| Peanuts | — | *Arachis hypogea* | 1 |
| Soyabean | — | *Glycine max* | 1.5 |
| Potatoes | — | *Solanum tuberosum* | 1.5 |
| Sunflowers | — | *Helianthus annus* | 0.5 |
| Carrots | — | *Daucus carota* | 0.5 |
| *Weeds* | | | |
| Yorkshire fog | — | *Holcus lanatus* | 6 |
| Chickweed | — | *Stellaria media* | 6 |
| Speedwell | — | *Veronica persica* | 6 |

Example 9

The composition of Example 5 was sprayed in an aqueous suspension in the field at the rates of the pyrazole compound given in the Table below post-emergence of the plant species listed. The herbicidal and plant growth regulant effect is shown on a scale from 0 to 10 on which 0 represents no effect and 10 complete kill.

| | | Effect | | |
|---|---|---|---|---|
| Species | Rate, kg per ha | 0.25 | 0.5 | 1 |
| Spring Barley | — *Hordeum vulgare* | 0.5 | 0.5 | 0.5 |
| Cleavers | — *Galium aparine* | 9.5 | 9.5 | 9.5 |
| Charlock | — *Sinapis arvensis* | 6 | 8.5 | 8.5 |
| Black Bindweed | — *Polygonum convolvulus* | 10 | 10 | 10 |
| Redshank | — *Polygonum persicaria* | 5.5 | 6 | 9 |

Example 10

The composition of Example 5 was sprayed in an aqueous suspension at 200 litres of liquid per hectare, at the rate of the pyrazole compound shown in the Table below, onto the following species:

Winter wheat (variety Bouquet) Zadoks 16, 22

Winter barley (variety M Otter) Zadoks 15, 23

Mayweed (a) Transplanted 5 cm across
(b) Sown 10 cm

Speedwell — branched 8—10 cm across

Cleavers — branched 8—10 cm across.

The experiments were conducted in a polythene tunnel and finally a glasshouse. Six weeks after the chemical treatment, percent growth reduction was assessed. The results are shown in the following Table:

| Rate kg/ha | Wheat (Bouquet) | Barley (M Otter) | Cleavers | Speedwell | Mayweed* |
|---|---|---|---|---|---|
| 3.0 | 0 | 10 | — | — | — |
| 1.0 | 0 | 7 | — | — | 53 |
| 0.5 | — | — | 85 | 80 | 15 |
| 0.25 | — | — | 60 | 65 | — |
| 0.25 | — | — | — | 30 | — |

*Mean of large and small plants

Example 11

The composition of Example 5 was sprayed in an aqueous tank mix with the other herbicides specified in the Table below onto the following species in a greenhouse:

Winter wheat (Bouquet) 16, 21 15 cm

Winter barley (M Otter) Well tillered, 15 cm

| | | |
|---|---|---|
| Mayweed | (a) | 6—10 leaves, 5—8 cm |
| | (b) | 8 leaves, 10 cm |
| Speedwell | — | branching, 5—8 cm |
| Cleavers | (a) | 2 branches, 10 cm |
| | (b) | 4—6 branches, 12 cm |
| Chickweed | (a) | Mainly 2 branches, 10—12 cm across |
| | (b) | Well-branched, 15 cm |

The rate of each active ingredient is shown in the Table, as are the percentage growth reductions of the species assessed 3 weeks after the chemical treatment.

| Treatment | Rate kg per ha | Wheat (Bouquet) | Barley (M Otter) | Cleavers | Speedwell | Chickweed | Mayweed |
|---|---|---|---|---|---|---|---|
| Compound + bentazone | 0.75 + 1.5 | 0 | 10 | | | | |
| " + " | 0.25 + 0.5 | 10 | 5 | 78 | 85 | 75 | 89 |
| " + " | 0.125 + 0.25 | | | 55 | 55 | 45 | 78 |
| Compound + bentazone | 1.5 + 1.5 | 5 | 5 | | | | |
| " + " | 0.5 + 0.5 | 5 | 0 | 78 | 80 | 68 | 97 |
| " + " | 0.25 + 0.25 | | | 65 | 70 | 58 | 80 |
| Compound + methoprotryne | 0.5 + 0.35 | 5 | 5 | 75 | 100 | 100 | 93 |
| " + " | 0.25 + 0.175 | | | 55 | 90 | 88 | 83 |
| Compound + metoxuron + simazine | 1.5 + 2.26 + 0.14 | 5 | 5 | | | | |
| " + " | 0.5 + 0.75 + 0.047 | 15 | 20 | 88 | 90 | 94 | 92 |
| " + " | 0.25 + 0.38 + 0.024 | | | 80 | 75 | 98 | 92 |

Example 12

The composition of Example 5 was sprayed in the autumn pre-emergence in an aqueous tank mix with the other herbicides specified in the Table below, onto plots in fields in which winter wheat or winter barley had been sown. The dose rate of the present compound and of the other herbicides, and the percentage average control of the weed species achieved by the mixtures compared to untreated plots, are shown. The assessments were made in the following June. There were 7 winter wheat and 2 winter barley trials.

| Treatment | Rate, kg per ha | % Weed Control | | | | | |
|---|---|---|---|---|---|---|---|
| | | Chickweed | Mayweed | Speedwell | Cleavers | Poppy | Black bindweed |
| Compound + metoxuron + simazine | 0.75 + 1.1 + 0.07 | 52 | 52 | 33 | 62 | 75 | 20 |
| Compound + trietazine | 0.75 + 0.6 | 45 | 13 | 63 | 38 | 90 | 58 |

Poppy is *Papaver rhoes.*

Example 13

The composition of Example 5 was sprayed in April or May pre-emergence either alone in an aqueous suspension, or in an aqueous tank mix with the other herbicides specified in the table below, onto plots in fields in which spring wheat (3 trials) or spring barley (3 trials) had been sown. The dose rate of the present compound and of the other herbicides is shown, as is the percentage average control which was achieved over the weed species compared to untreated plots. The assessments were made in late May or in June.

| Treatment | Rate, kg per ha | Weed Control | | | | | | | | | |
|---|---|---|---|---|---|---|---|---|---|---|---|
| | | Chickweed | Mayweed | Speedwell | Cleavers | Pale persicaria | Black bindweed | Mustard/charlock | Fathen | Shepherds purse | Annual nettle |
| Compound | 0.38 | 3 | 93 | 83 | 23 | 15 | 86 | 71 | 75 | 100 | 100 |
| ,, | 0.75 | 10 | 100 | 99 | 85 | 10 | 98 | 80 | 95 | 100 | 100 |
| Compound + metoxuron + simazine | 0.38 + 0.75 + 0.047 | 87 | 98 | 93 | 38 | 50 | 97 | 91 | 98 | 100 | 98 |
| ,, + ,, | 0.75 + 0.75 + 0.047 | 89 | 100 | 99 | 68 | 77 | 97 | 89 | 99 | 100 | 100 |
| ,, + ,, | 0.75 + 1.1 + 0.07 | 96 | 100 | 98 | 73 | 85 | 99 | 94 | 99 | 100 | 100 |
| Compound + pendimethalin | 0.38 + 0.38 | 84 | 100 | 99 | 50 | 42 | 68 | 59 | 98 | 100 | 100 |
| ,, + ,, | 0.75 + 0.38 | 95 | 100 | 99 | 53 | 60 | 97 | 77 | 99 | 100 | 98 |

In each case, the spring wheat or spring barley was not damaged.

Example 14

The composition of Example 5 was sprayed in April or May in an aqueous tank mix with the other herbicides specified in the table below post-emergence onto plots in fields in which winter wheat (5 trials) or winter barley (1 trial) was growing. The dose rate of the present compound and of the other herbicides is shown, as is the percentage average control which was achieved over the weed species compared to untreated plots. The assessments were made in May or June. The rate for CMPP is expressed in the conventional way in terms of the weight of equivalent acid.

| Treatment | Rate, kg per ha | Weed Control | | | |
|---|---|---|---|---|---|
| | | Chickweed | Cleavers | Mayweed | Speedwell |
| Compound + CMPP | | 74 | 97 | 92 | 100 |
| + bentazone | 0.5 + 2.5 + 0.2 | 70 | 98 | 92 | 100 |
| Compound + CMPP | | 69 | 89 | 91 | 100 |
| + bromoxynil | 0.5 + 2.5 + 0.4 | 79 | 96 | 99 | 100 |

In each case, the winter wheat or winter barley was not damaged.

Example 15

The composition of Example 5 was sprayed in May or June post-emergence in an aqueous tank mix with the other herbicides specified in the table below onto plots in fields in which spring barley was growing. Four trials were carried out. The dose rate of the present compound and of the other herbicides is shown, as is the percentage average control which was achieved over the weed species compared to untreated plots. The assessments were made in May or June. The rate for CMPP is expressed in the conventional way in terms of the weight of equivalent acid.

| Treatment | Rate, kg per ha | % Weed Control | | | | | | |
|---|---|---|---|---|---|---|---|---|
| | | Chickweed | Cleavers | Mayweed | Speedwell | Knotgrass | Field Pansy | Annual Nettle |
| Compound + CMPP | | 83 | 97 | 88 | 85 | 75 | 76 | 100 |
| + bentazone | 0.35 + 1.8 + 0.14 | 79 | 98 | 94 | 93 | 86 | 93 | 100 |
| Compound + CMPP | | 65 | 63 | 78 | 83 | 95 | 78 | 100 |
| + bromoxynil | 0.35 + 1.8 + 0.28 | 89 | 63 | 77 | 75 | 92 | 88 | 100 |

Knotgrass is *Polygonum aviculare.*
In each case, the spring barley was not damaged.

Example 16

The composition of Example 5 was sprayed in April post-emergence in an aqueous tank mix with the other herbicides specified in the table below onto plots in a field in which winter wheat was growing. The dose rate of the present compound and of the other herbicides is shown, as is the percentage average weed control which was achieved over the weed species compared to untreated plots. The assessments were made in May.

| | | % Weed Control | |
|---|---|---|---|
| Treatment | Rate, kg per ha | Speedwell | Cleavers |
| Compound + chlortoluron | 0.25 + 2.7 | 82 | 63 |
| | 0.5 + 2.7 | 88 | 87 |
| | 1.0 + 2.7 | 96 | 98 |
| Compound + isoproturon | 0.5 + 2.1 | 82 | 65 |

**Claims for the Contracting States: BE CH LI DE FR GB IT LU NL SE**

1. A compound which is 5-amino-4-cyano-1-(2,4,6-trichlorophenyl)pyrazole or a salt thereof.
2. 5-Amino-4-cyano-1-(2,4,6-trichlorophenyl)pyrazole.
3. A process for preparing a compound claimed in claim 1, which process comprises reacting 2,4,6-trichlorophenylhydrazine with a lower alkoxymethylenemalononitrile whose lower alkoxy group contains 1—4 carbon atoms.
4. A plant physiologically active composition comprising a compound claimed in claim 1 together with at least one material selected from surface active agents, carriers, antidotes, fertilizers, other plant growth regulants and other pesticides.
5. A composition according to claim 4 which contains a surface active agent, a solid carrier, a liquid carrier which is a hydrocarbon which boils within the range 130—270°C, an antidote, a fertilizer or another plant growth regulant or pesticide.
6. A herbicidal composition comprising a compound claimed in claim 1 together with another herbicide.
7. A composition according to claim 6 wherein the weight ratio of the compound claimed in claim 1 to the other herbicide is from 1:11 to 10:1.
8. A composition according to claim 6 or 7 wherein the other herbicide is 3-(3-chloro-4-methylphenyl)-1,1-dimethylurea.
9. A composition according to claim 6 or 7 wherein the other herbicide is 3-(4-isopropylphenyl)-1,1-dimethylurea.
10. A method of combating weeds at a locus infested or liable to be infested with them, or of regulating the growth of a plant at a locus at which the plant is growing or is to grow, which method comprises applying to the locus a weed-combating or plant growth regulant amount of the compound claimed in claim 1.

**Claims for the Contracting State: AT**

1. A process for preparing 5-amino-4-cyano-1-(2,4,6-tri-chlorophenyl)pyrazole or a salt thereof which process comprises reacting 2,4,6-trichlorophenyl-hydrazine with a lower alkoxymethylenemalononitrile whose lower alkoxy group contains 1—4 carbon atoms.
2. A plant physiologically active composition comprising 5-amino-4-cyano-1-(2,4,5-trichlorophenyl)pyrazole or a salt thereof together with at least one material selected from surface active agents, carriers, antidotes, fertilizers, other plant growth regulants and other pesticides.
3. A composition according to claim 2 which contains a surface active agent, a solid carrier, a liquid carrier which is a hydrocarbon which boils within the range 130—270°C, an antidote, a fertilizer or another plant growth regulant or pesticide.
4. A herbicidal composition comprising 5-amino-4-cyano-1-(2,4,6-trichlorophenyl)pyrazole or a salt thereof together with another herbicide.
5. A composition according to claim 4 wherein the weight ratio of the pyrazole compound to the other herbicide is from 1:11 to 10:1.
6. A composition according to claim 4 or 5 wherein the other herbicide is 3-(3-chloro-4-methylphenyl)-1,1-dimethylurea.
7. A composition according to claim 4 or 5 wherein the other herbicide is 3-(4-isopropylphenyl)-1,1-dimethylurea.

8. A method of combating weeds at a locus infested or liable to be infested with them, or of regulating the growth of a plant at a locus at which the plant is growing or is to grow, which method comprises applying to the locus a weed-combating or plant growth regulant amount of 5-amino-4-cyano-1-(2,4,6-trichlorophenyl) pyrazole or a salt thereof.

**Patentansprüche für die Vertragsstaaten: BE CH DE FR GB IT LI LU NL SE**

1. Eine Verbindung, nämlich 5-Amino-4-cyano-1-(2,4,6-trichlorphenyl)-pyrazol oder ein Salz hievon.

2. 5-Amino-4-cyano-1-(2,4,6-trichlorphenyl)-pyrazol.

3. Verfahren zur Herstellung einer Verbindung wie in Anspruch 1 beansprucht, welches Verfahren die Umsetzung von 2,4,6-Trichlorphenylhydrazin mit einem nied.Alkoxymethylenmalonnitril, dessen nied.Alkoxygruppe 1 bis 4 C-Atome enthält, umfaßt.

4. Pflanzenphysiologisch aktive Zusammensetzung, die eine in Anspruch 1 beanspruchte Verbindung zusammen mit mindestens einem Material ausgewählt unter oberflächenaktiven Mitteln, Trägern, Antidoten, Düngemitteln, anderen Pflanzenwachstumsregulatoren und anderen Schädlingsbekämpfungsmitteln enthält.

5. Zusammensetzung gemäß Anspruch 4, welche ein oberflächenaktives Mittel, einen festen Träger, einen flüssigen Träger, der ein Kohlenwasserstoff ist, welcher innerhalb des Bereiches von 130 bis 270°C siedet, ein Antidot, ein Düngemittel oder einen anderen Pflanzenwachstumsregulator oder ein anderes Schädlingsbekämpfungsmittel enthält.

6. Herbizide Zusammensetzung, die eine in Anspruch 1 beanspruchte Verbindung zusammen mit einem anderen Herbizid enthält.

7. Zusammensetzung gemäß Anspruch 6, worin das Gewichtsverhältnis der in Anspruch 1 beanspruchten Verbindung zum anderen Herbizid 1:11 bis 10:1 beträgt.

8. Zusammensetzung gemäß Anspruch 6 oder 7, worin das andere Herbizid 3-(3-Chlor-4-methylphenyl)-1,1-dimethylharnstoff ist.

9. Zusammensetzung gemäß Anspruch 6 oder 7, worin das andere Herbizid 3-(4-Isopropylphenyl)-1,1-dimethylnharnstoff ist.

10. Verfahren zum Bekämpfen von Unkraut an einer Stelle, die davon befallen ist oder dazu neigt, davon befallen zu werden, oder zum Regulieren des Wachstums einer Pflanze an einer Stelle, an der die Pflanze wächst oder wachsen soll, welches Verfahren das Aufbringen einer unkrautbekämpfenden oder pflanzenwachstumsregulierenden Menge der in Anspruch 1 beanspruchten Verbindung auf die Stelle umfaßt.

**Patentansprüche für den Vertragsstaat: AT**

1. Verfahren zur Herstellung von 5-Amino-4-cyano-1-(2,4,6-trichlorphenyl)-pyrazol oder eines Salzes hievon, welches Verfahren die Umsetzung von 2,4,6-Trichlorphenylhydrazin mit einem nied.Alkoxymethylenmalonnitril, dessen nied.Alkoxygruppe 1 bis 4 C-Atome enthält, umfaßt.

2. Pflanzenphysiologisch aktive Zusammensetzung, die 5-Amino-4-cyano-1-(2,4,5-trichlorphenyl)-pyrazol oder ein Salz hievon zusammen mit mindestens einem Material ausgewählt unter oberflächenaktiven Mitteln, Trägern, Antidoten, Düngemitteln, anderen Pflanzenwachstumsregulatoren und anderen Schädlingsbekämpfungsmitteln enthält.

3. Zusammensetzung gemäß Anspruch 2, welche ein oberflächenaktives Mittel, einen festen Träger, einen flüssigen Träger, der ein Kohlenwasserstoff ist, welcher innerhalb des Bereiches von 130 bis 270°C siedet, ein Antidot, ein Düngemittel oder einen anderen Pflanzenwachstumsregulator oder ein anderes Schädlingsbekämpfungsmittel enthält.

4. Herbizide Zusammensetzung, die 5-Amino-4-cyano-1-(2,4,6-trichlorphenyl)-pyrazol oder ein Salz hievon zusammen mit einem anderen Herbizid enthält.

5. Zusammensetzung gemäß Anspruch 4, worin das Gewichtsverhältnis der Pyrazolverbindung zum anderen Herbizid 1:11 bis 10:1 beträgt.

6. Zusammensetzung gemäß Anspruch 4 oder 5, worin das andere Herbizid 3-(3-Chlor-4-methylphenyl)-1,1-dimethylharnstoff ist.

7. Zusammensetzung gemäß Anspruch 4 oder 5, worin das andere Herbizid 3-(4-Isopropylphenyl)-1,1-dimethylharnstoff ist.

8. Verfahren zum Bekämpfen von Unkraut an einer Stelle, die davon befallen ist oder dazu neigt, davon befallen zu werden, oder zum Regulieren des Wachstums einer Pflanze an einer Stelle, an der die Pflanze wächst oder wachsen soll, welches Verfahren das Aufbringen einer unkrautbekämpfenden oder pflanzenwachstumsregulierenden Menge an 5-Amino-4-cyano-1-(2,4,6-trichlorphenyl)-pyrazol oder eines Salzes hievon auf die Stelle umfaßt.

**Revendications pour les Etats contractants: BE CH DE FR GB IT LI LU NL SE**

1. Composé qui est le 5-amino-4-cyano-1-(2,4,6-trichlorophényl)pyrazole ou un de ses sels.

2. Le 5-amino-4-cyano-1-(2,4,6-trichlorophényl)pyrazole.

3. Procédé pour préparer un composé suivant la revendication 1, lequel procédé comprend la réaction de la 2,4,6-trichlorophénylhydrazine avec un alcoxy(inférieur)méthylènemalononitrile dont le radical alcoxy inférieur compte 1—4 atomes de carbone.

4. Composition active en physiologie végétale comprenant un composé suivant la revendication 1 outre au moins une substance choisie entre les agents surfactifs, les excipients, les antidotes, les fertilisants, d'autres régulateurs de la croissance des plantes et d'autres pesticides.

5. Composition suivant la revendication 4, qui contient un agent surfactif, un excipient solide, un excipient liquide qui est un hydrocarbure qui bout dans l'intervalle de 130—270°C, un antidote, un fertilisant ou un autre régulateur de la croissance des plantes ou pesticide.

6. Composition herbicide comprenant un composé suivant la revendication 1 outre un autre herbicide.

7. Composition suivant la revendication 6, dans laquelle le rapport pondéral du composé suivant la revendication 1 à l'autre herbicide est de 1:11 à 10:1.

8. Composition suivant la revendication 6 ou 7, dans laquelle l'autre herbicide est la 3-(3-chloro-4-méthylphényl)-1,1-diméthylurée.

9. Composition suivant la revendication 6 ou 7, dans laquelle l'autre herbicide est la 3-(4-isopropylphényl)-1,1-diméthylurée.

10. Procédé de lutte contre la végétation indésirable en un lieu qui en est infesté ou susceptible d'en être infesté ou de régulation de la croissance d'une plante en un lieu où la plante est en croissance ou doit croître, lequel procédé comprend l'application en ce lieu du composé suivant la revendication 1 en une quantité combattant la végétation indésirable ou régulant la croissance de la plante.

**Revendications pour l'Etat contractant: AT**

1. Procédé pour préparer le 5-amino-4-cyano-1-(2,4,6-trichlorophényl)pyrazole ou un de ses sels, lequel procédé comprend la réaction de la 2,4,6-trichlorophénylhydrazine avec un alcoxy(inférieur)-méthylènemalononitrile dont le radical alcoxy inférieur compte 1—4 atomes de carbone.

2. Composition active en physiologie végétale comprenant du 5-amino-4-cyano-1-(2,4,6-trichlorophényl)-pyrazole ou un de ses sels outre au moins une substance choisie entre les agents surfactifs, les excipients, les antidotes, les fertilisants, d'autres régulateurs de la croissance des plantes et d'autres pesticides.

3. Composition suivant la revendication 2 qui contient un agent surfactif, un excipient solide, un excipient liquide qui est un hydrocarbure qui bout dans l'intervalle de 130—270°C, un antidote, un fertilisant ou un autre régulateur de la croissance des plantes ou pesticide.

4. Composition herbicide comprenant du 5-amino-4-cyano-1-(2,4,6-trichlorophényl)pyrazole ou un de ses sels outre un autre herbicide.

5. Composition suivant la revendication 4, dans laquelle le rapport pondéral du pyrazole à l'autre herbicide est de 1:11 à 10:1.

6. Composition suivant la revendication 4 ou 5, dans laquelle l'autre herbicide est la 3-(3-chloro-4-méthylphényl)-1,1-diméthylurée.

7. Composition suivant la revendication 4 ou 5, dans laquelle l'autre herbicide est la 3-(4-isopropylphényl)-1,1-diméthylurée.

8. Procédé de lutte contre la végétation indésirable en un lieu qui en est infesté ou susceptible d'en être infesté ou de régulation de la croissance d'une plante en un lieu où la plante est en croissance ou doit croître, lequel procédé comprend l'application en ce lieu d'une quantité de 5-amino-4-cyano-1-(2,4,6-trichlorophényl)pyrazole ou d'un de ses sels en une quantité combattant la végétation indésirable ou régulant la croissance de la plante.